# EUROPEAN PATENT APPLICATION

(11) **EP 2 422 835 A1**
(43) Date of publication of application: **29.02.2012**
(21) Application number: 10767097.8
(22) Date of filing: 21.04.2010
(51) Int. Cl.: A61M 25/02, A61M 25/00

(54) **CATHETER, CATHETER SECURING DEVICE, CATHETER KIT, AND METHOD OF MANUFACTURING CATHETER**

(30) Priority: 22.04.2009 JP 2009104530
(71) Applicant: Olympus Corporation, Shibuya-ku Tokyo 151-0072 (JP)
(72) Inventor: KOSEKI, Hiroaki, Tokyo 151-0072 (JP)
(74) Representative: Schicker, Silvia
(86) International application number: PCT/JP2010/057098
(87) International publication number: WO 2010/123040

(57) **Abstract**

Provided are an elongated tubular catheter (19) having at least one drug discharge hole in a side and having a penetration hole (11) which is a first engaging structure in a distal end part closer to a distal end side than the drug discharge hole; and a catheter fixture (20) that is separated from the catheter (19) and has an engaging groove (26) which is a second engaging structure which engages with the penetration hole (11). The catheter fixture (20) is fixed to a distal end part of the catheter (19) when the penetration hole (11) and engaging groove (26) engage, and is wider than the diameter of the catheter (19) in a direction perpendicular to an axis of the catheter (19) in a fixed state.

## Description

### Field

The present invention relates to a catheter inserted inside the body of mammals including human beings, a catheter fixture, a catheter kit and a catheter manufacturing method.

### Background

Conventionally, a catheter is inserted into the body of a mammal including human beings to, for example, administer a drug inside the body utilizing this catheter. In this case, the elongated tubular catheter is moved in its axial direction and inserted in tissues of the body (see, for example, Patent Literature 1).

### Citation List

### Patent Literature

Patent Literature 1: Japanese National Publication of International Patent Application No. 2003-504132

### Summary

### Technical Problem

The elongated tubular catheter is likely to be misaligned in the axial direction after being inserted in the body tissues, and therefore there are cases where this catheter does not abut on the body tissues, or drug administration holes provided at a distal end part of the catheter are misaligned and therefore a drug cannot be administered to a desired position.

In view of the foregoing, it is therefore an object of the present invention to provide a catheter, a catheter fixture, a catheter kit and a catheter manufacturing method, capable of reducing misalignment of the catheter with respect to the body tissues, particularly, the organ of a treatment target.

### Solution to Problem

To solve the problem as described above and achieve the object, a catheter kit according to the present invention includes an elongated tubular catheter having at least one drug discharge hole in a side of the catheter, the catheter having a first engaging structure in a distal end part closer to a distal end side than the drug discharge hole; and a catheter fixture that is separated from the catheter, the catheter fixture having a second engaging structure that engages with the first engaging structure, the catheter fixture being fixed to the distal end part of the catheter with when the first engaging structure and the second engaging structure engage, the catheter fixture being wider than a diameter of the catheter in a direction perpendicular to an axis of the catheter in a fixed state.

In the catheter kit according to the present invention, the catheter is used to administer a drug to a drug administration target in an organ, and when the catheter penetrates the organ and the catheter fixture is in a position to abut on the organ in a state where the catheter fixture is fixed to the distal end part, the drug discharge hole is provided in a position adjusted in advance such that the drug discharge hole is positioned inside the drug administration target.

In the catheter kit according to the present invention, the catheter has a flange in a position closer to a proximal end side than a position of at least one drug discharge hole.

In the catheter kit according to the present invention, the catheter is used to administer a drug to a drug administration target in an organ, and when the catheter penetrates the organ and the catheter fixture is in a position to abut on the organ in a state where the catheter fixture is fixed to the distal end part, the flange is provided in a position on the catheter adjusted in advance such that the flange abuts on the organ.

In the catheter kit according to the present invention, the catheter fixture has a protecting part that prevents a tip of the catheter and an organism from contacting, in a part positioned on an extension of an axis line on a distal end side of the catheter in a state where the catheter fixture is fixed to the distal end part of the catheter.

In the catheter kit according to the present invention, the catheter is for puncturing an organ, and the catheter fixture has a flange that abuts on the organ, in a part positioned closer to a proximal end side of the catheter than the second engaging structure in a state where the catheter fixture is fixed to the distal end part of the catheter.

In the catheter kit according to the present invention, the catheter fixture includes a base member and a slider having the second engaging structure, and when the catheter and the catheter fixture are in predetermined positions, the slider slides with respect to the base member such that the first engaging structure of the catheter and the second engaging structure of the catheter fixture engage.

In the catheter kit according to the present invention, the slider slides with respect to the base member according to a manipulation from an outside of a body.

In the catheter kit according to the present invention, the manipulation from the outside of the body is a manipulation of a wire for sliding the slider.

In the catheter kit according to the present invention, the manipulation from the outside of the body is a manipulation of moving a fluid for sliding the slider, with respect to the catheter fixture.

In the catheter kit according to the present invention, the catheter fixture has an elongated wire rod, the second engaging structure is a gripping part that grips the first engaging structure, and the gripping part is provided at a distal end of the wire rod and is manipulated at a proximal end side of the wire rod.

In the catheter kit according to the present invention, the catheter has a wire rod that stretches from a distal end part, and the first engaging structure is provided in the wire rod.

In the catheter kit according to the present invention, a plurality of wire rods are provided, and the first engaging structure is provided in each of the plurality of wire rods.

In the catheter kit according to the present invention, on a surface of the catheter, a pattern which serves as an indication to determine a punching position of the drug discharge hole is displayed.

In the catheter kit according to the present invention, the distal end part can be separated from the catheter.

In the catheter kit according to the present invention, the catheter can be separated at a distal end side of the flange.

In the catheter kit according to the present invention, the catheter has a bent part closer to a proximal end side than the drug administration hole.

A catheter kit according to the present invention includes an elongated tubular catheter having at least one drug discharge hole in a side, having a first engaging structure in a distal end part closer to a distal end side than the drug discharge hole, and having a first flange that is a flange provided in a position closer to a proximal end side than a position of the drug discharge hole; and a catheter fixture that is separated from the catheter, the catheter fixture having a second engaging structure that engages with the first engaging structure, and having a second flange that is a flange provided in a position closer to the distal end side than a position of the drug discharge hole.

In the catheter kit according to the present invention, the second engaging structure forms the second flange.

A catheter according to the present invention is for puncturing an organ to administer a drug to a drug administration target inside the organ, and includes an elongated tubular body, the body having at least one drug discharge hole in a side, the body having a flange closer to a proximal end side than the drug discharge hole, wherein when the catheter punctures the organ along a route determined in advance and the flange is in a position to abut on the organ, the drug discharge hole is provided in a position generated in advance such that the drug administration hole is positioned inside the drug administration target.

A catheter according to the present invention includes an elongated tubular catheter body having at least one drug discharge hole; and at least one wire rod capable of being transitioned from a first state where the entire wire rod is accommodated in an internal passage inside the catheter body to a second state where a distal end part projects from the internal passage on a side closer to a distal end side than the drug discharge hole, the wire rod fixing the catheter body to a body tissue in the second state.

A catheter fixture according to the present invention is separated from a catheter for puncturing an organ to administer a drug to a drug administration target inside the organ, and includes a second engaging structure that engages with a first engaging structure formed at a distal end part of the catheter, the catheter fixture being fixed to the distal end part of the catheter when the first engaging structure and the second engaging structure engage, the catheter fixture being wider than a diameter of the catheter in a direction perpendicular to an axis of the catheter in a fixed state.

A method of manufacturing a catheter according to the present invention is a method of manufacturing a catheter to be inserted in an organ to administer a drug to a drug administration target inside the organ, and includes a target position acquiring step of acquiring positions of the organ and the drug administration target using an in-vivo observation device; a determining step of determining an insertion route of the catheter to the organ, based on the positions of the organ and the drug administration target acquired in the target position acquiring step; and a hole forming step of forming a drug discharge hole in a side of the catheter which meets the drug administration target, based on the positions of the organ and the drug administration target and the determined insertion route. Advantageous Effects of Invention

The present invention includes an elongated tubular having at least one drug discharge hole in a side and having a first engaging structure in a distal end part closer to a distal end side than the drug discharge hole; and a catheter fixture having a second engaging structure which is formed separately from the catheter and engages with the first engaging structure, which is fixed to a distal end part of the catheter when the first engaging structure and the second engaging structure engage, and which is wider than a diameter of the catheter in a direction perpendicular to an axis of the catheter in a fixed state, so that it is possible to reduce misalignment of the catheter with respect to the organ of a treatment target.

### Brief Description of Drawings

FIG. 1 is a schematic view schematically illustrating an organ and a tumor to which a catheter according to a first embodiment of the present invention is inserted.
FIG. 2 is a view illustrating a manufacturing process of a catheter according to the first embodiment of the present invention.
FIG. 3 is a view illustrating an example of a preparation process of a catheter illustrated in FIG. 2.
FIG. 4 is an explanatory view illustrating that a catheter is fixed to an organ by means of a catheter and catheter fixture according to the first embodiment of the present invention.
FIG. 5 is a view illustrating an example of separation process of a catheter and catheter fixture fixed to an organ.
FIG. 6 is a schematic view illustrating a separating position of a catheter and catheter fixture according to the first embodiment of the present invention.
FIG. 7 is a view illustrating a configuration of a catheter fixture according to a second embodiment of the present invention.
FIG. 8 is a schematic view illustrating a separating position of a catheter and catheter fixture according to the second embodiment of the present invention.
FIG. 9 is a perspective view illustrating a configuration of a catheter fixture according to a third embodiment of the present invention.
FIG. 10 is a perspective view illustrating a configuration of a catheter fixture according to a modification of the third embodiment of the present invention.
FIG. 11 is a schematic view illustrating a configuration and fixing process of a catheter and catheter fixture according to a fourth embodiment of the present invention.
FIG. 12 is a schematic view illustrating a configuration of a gripping part according to a first modification of the fourth embodiment of the present invention.
FIG. 13 is a schematic view illustrating a configuration of a gripping part according to a second modification of the fourth embodiment of the present invention.
FIG. 14 is a schematic view illustrating a configuration and fixing process of a catheter and catheter fixture according to a fifth embodiment of the present invention.
FIG. 15 is a schematic view illustrating a configuration of a catheter and catheter fixture according to a sixth embodiment of the present invention.
FIG. 16 is a schematic view illustrating a configuration of a catheter and catheter fixture according to a modification of the sixth embodiment of the present invention.
FIG. 17 is a schematic view illustrating a configuration and fixing process of a catheter and catheter fixture according to a seventh embodiment of the present invention.
FIG. 18 is a schematic view illustrating a configuration of a catheter according to a modification of the seventh embodiment of the present invention.
FIG. 19 is a schematic view illustrating a configuration and fixing process of a catheter and catheter fixture according to an eighth embodiment of the present invention.
FIG. 20 is a schematic view illustrating a configuration of a catheter and catheter fixture according to a ninth embodiment of the present invention. Description of Embodiments

Hereinafter, a catheter, a catheter fixture, a catheter kit and a catheter manufacturing method according to embodiments of the present invention will be described with reference to the drawings.

### First Embodiment

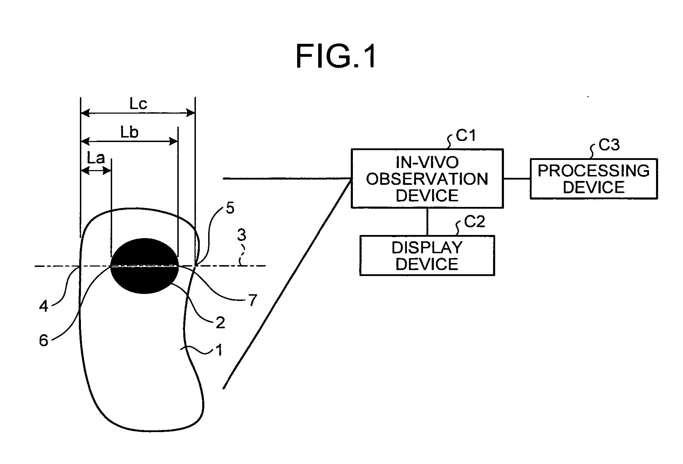

First, a catheter and a catheter manufacturing method will be described with reference to FIG. 1 to FIG. 3. FIG. 1 illustrates a state where there is a tumor 2 of a drug administration target inside an organ 1 of the body. Meanwhile, an operator acquires shapes and positions of the organ 1 and tumor 2 using an in-vivo observation device C1 such as an MRI, CT, roentgen device or ultrasonic detector.

Then, the operator specifies an insertion route 3 for administering a drug to the tumor 2 based on the shapes and positions of the organ 1 and tumor output on a display device C2 connected to the in-vivo observation device C1. This insertion route 3 may be specified automatically using a processing device C3 connected to the in-vivo observation device C1, or may be specified arbitrarily on a display screen according to an operation of the display device C2 by the operator.

Then, the processing device C3 calculates a parameter showing relative positions of the organ 1 and tumor 2 along the insertion route 3, based on the specified insertion route 3. For example, a distance La between a projecting position 4 of the catheter from the organ 1 and a projecting position 6 of the catheter from the tumor 2, a distance Lb between the projecting position 4 of the catheter from the organ 1 and an insertion position 7 of the catheter to the tumor 2 and a distance Lc between the projecting position 4 of the catheter from the organ 1 and an insertion position 5 of the catheter to the organ 1 are calculated. In addition, these parameters may be calculated by the operator depending on display content on the display screen of the display device C2.

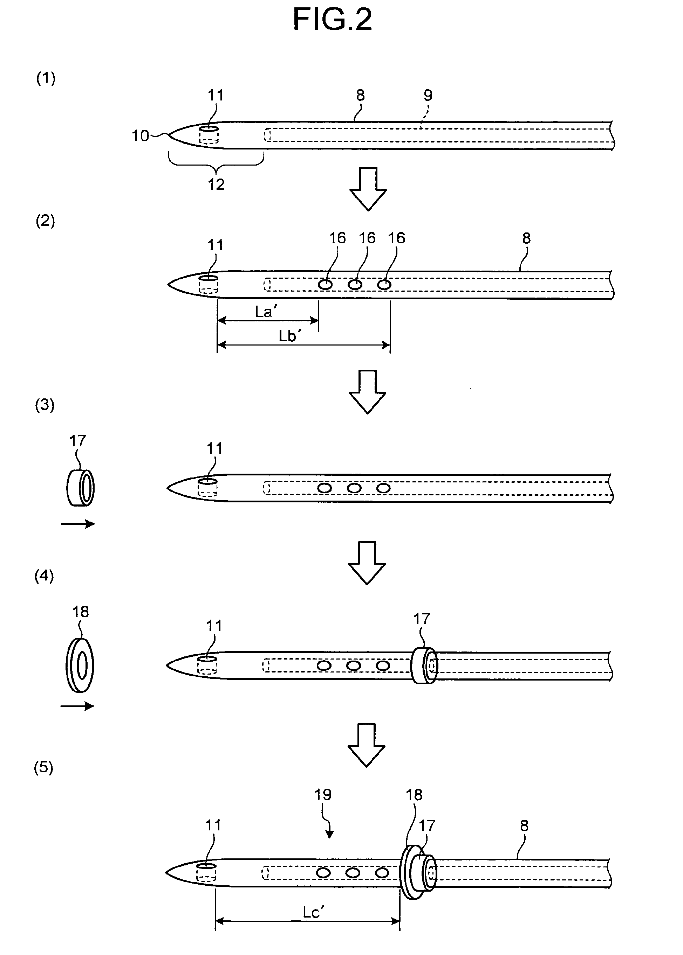

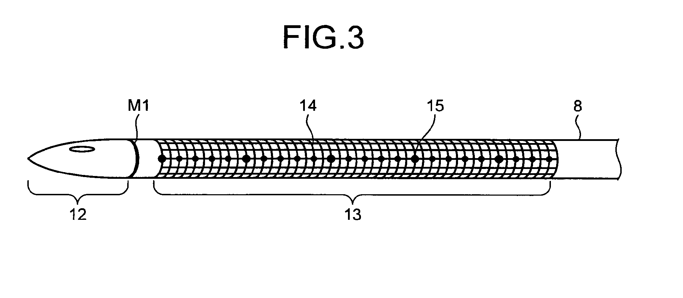

Meanwhile, as illustrated in (1) of FIG. 2, a catheter body 8 has an elongated, cylindrical shape having a sharp tip 10, and an internal passage 9 in which, for example, a drug flows. Further, a penetration hole 11 which is a first engaging structure is provided at a distal end part 12. In addition, as illustrated in FIG. 3, with the catheter body 8, mark lines 14 and scales 15 which serve as an indication for a punching operation are provided in a punching area 13 in which drug discharge holes will be formed. Further, a mark M1 is provided on a proximal end side of the distal end part 12, and serves as an indication upon an operation of separating only the distal end part 12.

As illustrated in (1) of FIG. 2, with the catheter body 8 in which the penetration hole 11 and internal passage 9 are formed, one or a plurality of drug discharge holes 16 are punched in the side of the catheter body 8 as illustrated in (2) of FIG. 2 based on the above parameters to manufacture a catheter suitable for the organ 1 and tumor 2 for which the positional relationship along the insertion route 3 is learned. With this punching, while a tube wall is penetrated in front of the catheter body 8 using, for example, a reamer, the tube wall ahead of the internal passage 9 is not penetrated. In this case, the drug discharge holes 16 are positioned using the mark lines 14 and scales 15 illustrated in FIG. 3. In addition, when the catheter is inserted and positioned in the organ 1, the positions of the drug discharge holes 16 are determined such that the drug discharge holes 16 are positioned inside the tumor 2.

In (2) of FIG. 2, three drug discharge holes 16 are punched in the side of the catheter body 8. That is, the inner wall of the penetration hole 11 at the catheter distal end on the proximal end side meets the projecting position 4 of the catheter from the organ 1, a distal end rim of the drug discharge hole closest to the distal end side meets the projecting position 6 of the catheter from the tumor 2, and the proximal end rim of the drug discharge hole closest to the proximal end side meets the insertion position 7 of the catheter to the tumor 2. Further, the distance La illustrated in FIG. 1 is equal to a distance La' between the inner wall of the penetration hole 11 of the catheter distal end on the proximal end side and the distal end rim of the drug discharge hole closest to the distal end side. Further, the distance Lb illustrated in FIG. 1 is equal to a distance Lb' between the inner wall of the penetration hole 11 of the catheter distal end on the proximal end side and the proximal end rim of the drug discharge hole closest to the proximal end side. That is, based on La = La' and Lb = Lb', the positions of the drug discharge holes 16 are within the range of Lb'-La'. By providing the drug discharge holes 16 within the range of this Lb'-La', the drug discharge holes 16 are positioned inside the tumor 2.

In addition, taking deformation or a margin of the organ 1 into account, the relationships of La = La' and Lb = Lb' may be intentionally changed. Further, the positions of the drug administration holes 16 vary depending on a treatment policy of, for example, concentrating administration of a drug on the center of the tumor 2. In any case, the drug discharge holes 16 are formed in positions from which a drug is administered inside the tumor 2, based on the above parameters.

Then, the operator fixes a flange 18 to the catheter body 8. In a state where the catheter is inserted in the organ 1, this flange 18 abuts on the surface of the organ 1 at the insertion position, and, upon this abuttal, all drug discharge holes 16 need to be positioned inside the tumor 2. Hence, the flange 18 abuts on the organ 1 at a position at which all drug discharge holes 16 are positioned inside the tumor 2. In this case, the fixing position of the flange 18 is closer to the proximal end side than the drug discharge hole 16 closest to the proximal end side among the drug discharge holes 16 formed in the catheter body 8.

More specifically, as illustrated in (3) of FIG. 2, a ring-shaped flange fixing member 17 for fixing the flange 18 to the catheter body 8 is first inserted from the catheter distal end side. The flange fixing member 17 after the insertion is adhered and fixed to the proximal end side of the catheter to function as a stopper at a position at which the flange 18 is positioned (see (4) of FIG. 2). Further, as illustrated in (4) of FIG. 2, the flange 18 is inserted in the catheter body 8 from the distal end side of the catheter, and is adhered and fixed to the flange fixing member 17. By this means, as illustrated in (5) of FIG. 2, a catheter 19 having the catheter body 8 and flange 18 is formed.

Meanwhile, a distance Lc' between the inner wall of the penetration hole 11 of the catheter distal end part on the proximal end side and the surface of the flange 18 on the distal end side (abutting surface with respect to the organ 1) is equal to the distance Lc illustrated in FIG. 1. By this means, by inserting the catheter 19 until the flange 18 abuts on the organ 1, it is possible to position all drug discharge holes 16 inside the tumor 2.

Although the catheter 19 can be manufactured like a custom-made catheter in this way, this is not the case when the catheters 19 are mass-produced according to the specification set in advance. Meanwhile, the catheter 19 has the drug administration holes 16 and flange 18 in the catheter body 8 as described above.

In addition, the catheter 19 is preferably sterilized.

The catheter 19 manufactured in this way is inserted in the organ 1 along the insertion route 3 illustrated in FIG. 1. The distal end part of the catheter 19 projecting from this organ 1 is fixed by a catheter fixture 20.

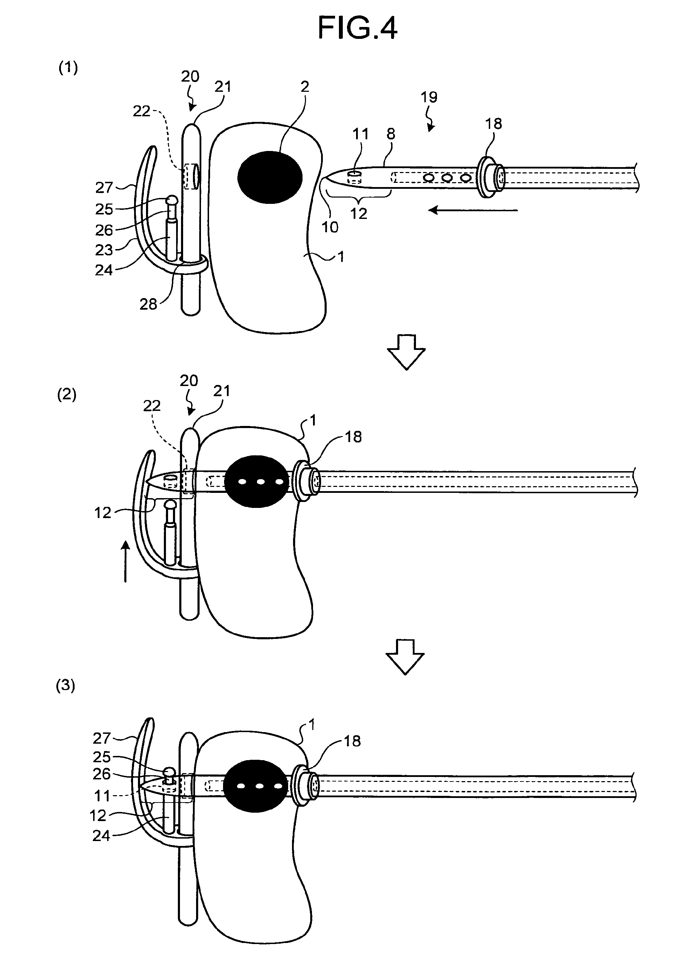

As illustrated in (1) of FIG. 4, the catheter fixture 20 has a slide bar 21 and a slider 23 which are base members. The slide bar 21 has a penetration hole 22 having a size which allows the distal end part 12 of the catheter 19 to pass therethrough. By contrast with this, the slider 23 has a shape bent in a nearly L-shape, and has in a lateral portion of the L-shape a penetration hole 28 through which the slide bar 21 penetrates. The slider 23 can move in the axial direction of the slide bar 21 through the penetration hole 28. A lock bar 24 is provided to stand near the penetration hole 28, an engaging groove 26 which is a second engaging structure engaging with the first engaging structure is formed on the upper part of the lock bar 24, and an elastic ball 25 is provided at the distal end of the engaging groove 26. Further, in the vertical portion of the L-shape, a protecting part 27 is formed which prevents the tip 10 of the catheter 19 from damaging other organs and tissues.

Further, positioning of the catheter 19 and catheter fixture 20 will be described with reference to FIG. 4. As illustrated in (2) of FIG. 4, when the catheter 19 is inserted until the flange 18 of the catheter 19 abuts on the surface of the organ 1, the catheter 19 penetrates the organ 1 and tumor 2 and the distal end part 12 projects on the opposite side of the organ 1. Hereinafter, the catheter fixture 20 is positioned in a position at which the distal end part 12 of the catheter 19 passes the penetration hole 22 of the slide bar 21, and the distal end part 12 penetrates the penetration hole 22. Then, when the slider 23 is moved upward along the axis of the slide bar 21, as illustrated in (3) of FIG. 4, the front end portion of the lock bar 24 is inserted in the penetration hole 11 and the elastic ball 25 has a greater diameter than the penetration hole 11 and elastically deforms and passes the penetration hole 11. As a result, the engaging groove 26 engages and positions the lock bar 24 and catheter 19, respectively. Further, the catheter 19 and catheter fixture 20 are fixed fast across the organ 1.

Meanwhile, the positional relationship of the catheter 19 and organ 1 is determined by the catheter fixture 20 on the distal end side, and is determined by the flange 18 on the proximal end side. Consequently, the relative positional relationship of the catheter 19 and organ 1 is fixed by the catheter fixture 20 and flange 18, and the catheter 19 is not misaligned in the axial direction with respect to the organ 1.

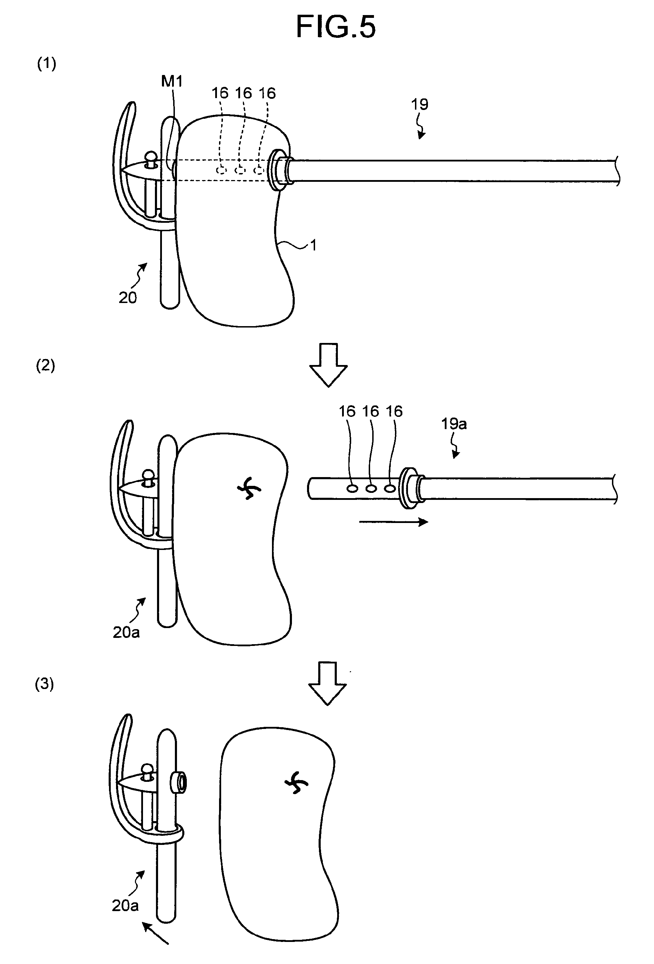

Then, when administration of a drug is finished through the catheter 19, the operator removes the catheter 19 and catheter fixture 20 to the outside of the body. That is, as illustrated in (1) of FIG. 5, for example, the catheter is cut at a mark M1 portion which serves as an indication of the mark M1 formed between the distal end part 12 of the catheter 19 and punching area 13. Further, as illustrated in (2) of FIG. 5, a catheter 19a from which the distal end part 12 is separated off is pulled out from the organ 1. Further, as illustrated in (3) of FIG. 5, a catheter fixture 20a engaged with the distal end part 12 is pulled out.

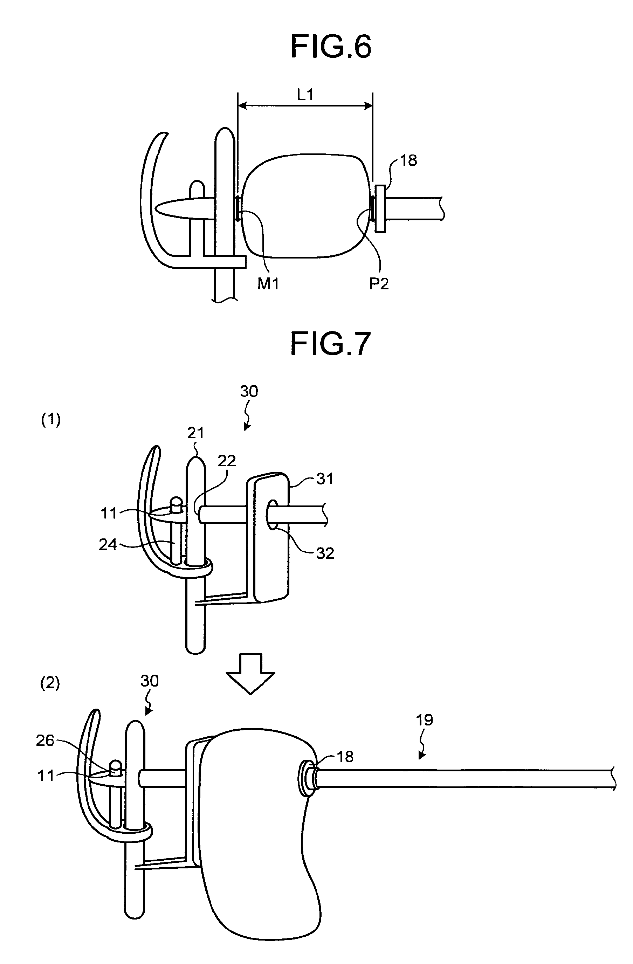

In addition, the separating position is not limited to the position of the mark M1, and the catheter may be separated at a position P2 on the organ 1 side of the flange 18 as illustrated in FIG. 6.

### Second Embodiment

Next, a second embodiment of the present invention will be described with reference to FIG. 7. As illustrated in (1) of FIG. 7, with this second embodiment, a catheter fixture 30 is formed by further providing a flange 31 to the above catheter fixture 20 on the organ 1 side. Although, with the first embodiment, the slide bar 21 has a function of a flange on the distal end side with respect to the organ 1, even when this slide bar 21 is not arranged near the organ 1 side, it is possible to reliably fix the catheter 19 to the organ 1 by providing a dedicated flange 31 on the organ 1 side. This flange 31 is provided with a penetration hole 32 through which the catheter 19 projecting from the organ 1 passes, and the catheter 19 passes this penetration hole 32 and penetration hole 22. In this case, when the flange 31 is provided, the positions of the drug discharge holes 16 on the catheter 19 need to be changed. In (2) of FIG. 7, illustrated is a mode of use of the catheter fixture 30 according to the second embodiment.

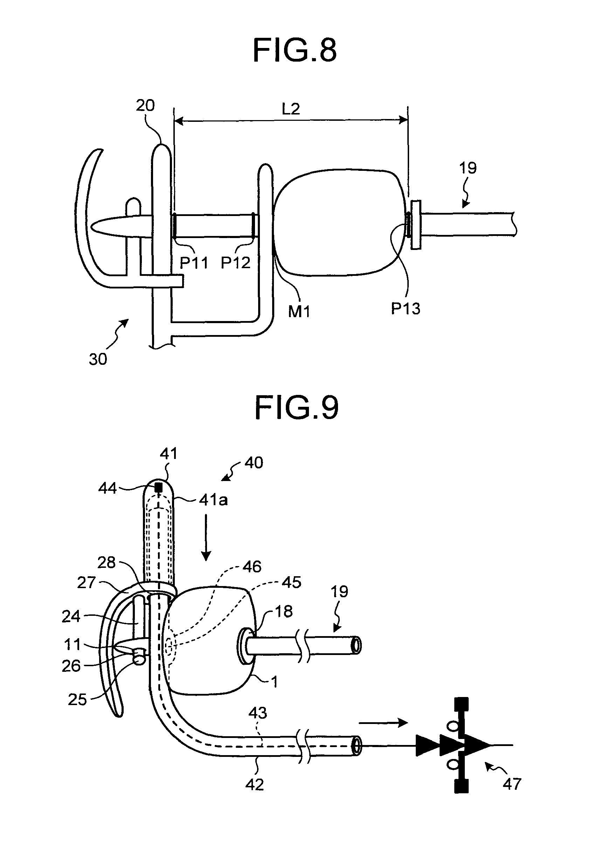

FIG. 8 is a view describing a separating position of the catheter 19 and catheter fixture 30 according to this second embodiment. As illustrated in FIG. 8, the catheter can be separated at the position of the mark M1 similar to the first embodiment. Further, it is possible to separate the catheter at a position P13 on the organ 1 side of the flange 18. In addition, the catheter may be separated at a position P12 on the distal end side of the penetration hole 32 of the flange 31 or at a position P11 on the proximal end side of the penetration hole 22 of the slide bar 20. When the catheter 19 is removed, if the catheter is separated at the position P11, the distance which the catheter 19 passes through the organ 1 is the distance L2 and is greater than the distance L1 illustrated in FIG. 6, and therefore the catheter is preferably separated at the positions P12, P13 or the position of the mark M1 to reduce invasion.

### Third Embodiment

Next, a third embodiment of the present invention will be described. With the third embodiment, engagement of the first engaging structure and second engaging structure can be manipulated from the outside of the body. FIG. 9 is a perspective view illustrating a configuration of a catheter fixture according to the third embodiment of the present invention. Although a catheter fixture 40 illustrated in FIG. 9 has a slider 41 having a shape bent in a nearly L-shape similar to the catheter fixture 20, with this slider 41, a cylindrical cap 41a into which a tube 42 is inserted is formed in the lateral portion of the L-shape. In the tube 42, a wire 43 which extends from the cap 41a to the outside of the body is inserted. A latch part 44 at the tip of the wire 43 is embedded in the bottom of the tube 41a and latched. Hence, when the operator pulls the wire 43 from the outside of the body, the cap 41a slides on the surface of the tube 42, the entire slider 41 moves downward as illustrated in FIG. 9 and the engaging groove 26 of the lock bar 24 engages with the penetration hole 11.

In addition, near the distal end of the tube 42 and on the insertion route 3 of the catheter 19, the tube 42 has a holding part 46 in which a penetration hole 45 through which the catheter 19 penetrates is formed. Further, the other end of the wire 43 forms a ratchet mechanism 47 which is disposed outside the body or the surface of the body, and this ratchet mechanism 47 fixes the position of the wire 43 in one direction. Further, this ratchet mechanism 47 can cancel the engaged state of the catheter 19 and catheter fixture 40 by canceling the ratchet operation of the ratchet mechanism 47 and pushing the wire 43 toward the distal end side. In addition, similar to the slide bar 21 according to the first embodiment, the tube 42 has the function of the flange which abuts on the organ 1.

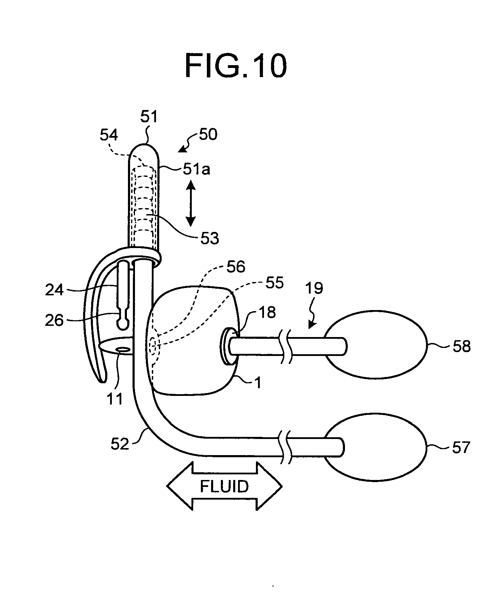

Further, as illustrated in FIG. 10, a configuration is possible where, instead of the wire 43, a slider 51 is movable bidirectionally by discharging and suctioning a fluid. That is, the distal end of a tube 52 corresponding to the tube 42 is inserted in a cap 51a of the slider 50, and, at this distal end, a stretching part 53 is formed which is stretched in the cap 51a by a fluid discharged and suctioned in the internal passage. The other end of the tube 52 is provided with a fluid pump 57 which discharges and suctions a fluid, and the fluid pump 57 discharges and suctions the fluid in and from the tube 52 to stretch the stretching part 53. The distal end of the stretching part 53 is latched with the cap 51a at a latch part 54 in the cap 51a. Following stretching of this stretching part 53, when the cap 51a moves in up and down directions as illustrated in FIG. 10, the entire slider 51 moves in the up and down directions and the engaging groove 26 of the lock bar 24 engages or disengages with the penetration hole 11. Further, this engaged state is maintained by stopping the flow of the fluid by the fluid pump 57.

The state where the engaging groove 26 of the lock bar 24 engages with the penetration hole 11 is a state where the catheter 19 is fixed to the organ 1, and, in this state, a drug is administered to the tumor inside the organ 1 from a drug administration pump 58 connected to the proximal end side of the catheter 19 through the drug discharge holes 16. In addition, similar to FIG. 9, near the distal end of the tube 52 and on the insertion route 3 of the catheter 19, the tube 52 has a holding part 56 in which a penetration hole 55 through which the catheter 19 penetrates is formed.

With this third embodiment, the catheter fixture 40 or 50 and catheter 19 can be engaged and disengaged from the outside of the body by moving the slider 41 or 51, that is, the catheter fixture 40 or 50 using the cap 41a or 51a and tube 42 or 52 and using the wire 43 or fluid in this tube 42 or 52, so that it is possible to easily exchange the catheter 19 without opening the abdominal cavity again.

### Fourth Embodiment

Next, a fourth embodiment of the present invention will be described. Although, with the above first to third embodiments, the catheter fixture and catheter are disposed by opening the abdominal cavity, with this fourth embodiment, the catheter 19 can be inserted and fixed by, for example, a manipulation using a laparoscope.

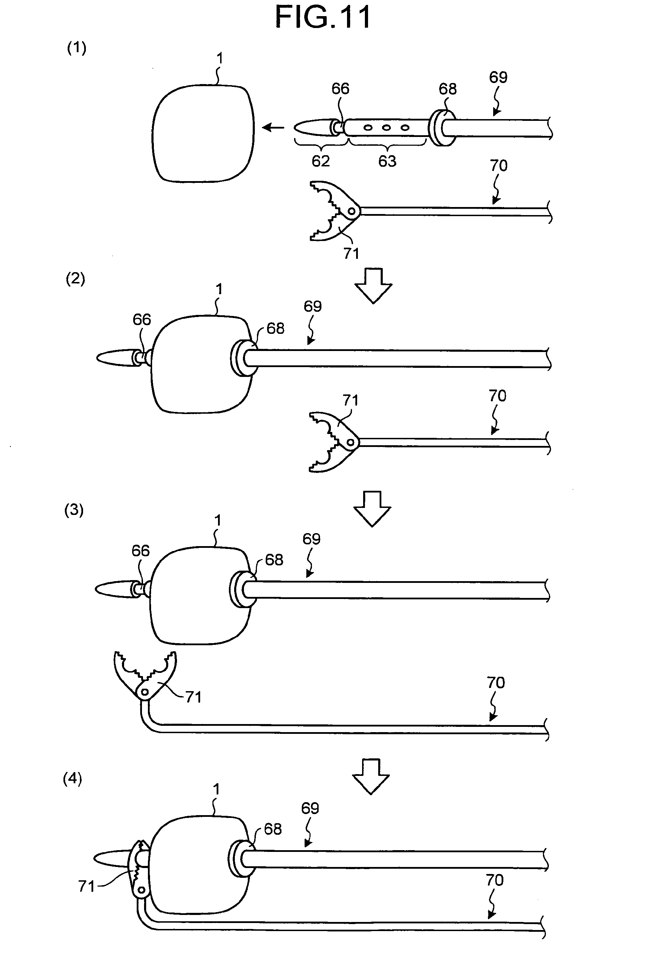

That is, as illustrated in (1) of FIG. 11, a catheter 69 has an engaging groove 66 which is a first engaging structure, in a proximal end part of a distal end part 62 corresponding to the distal end part 12 according to the first embodiment, drug discharge holes 16 formed in a punching area 63 and a flange 68 corresponding to the flange 18 according to the first embodiment. A catheter fixture 70 has at its distal end a gripping part 71 such as a pair of forceps which grips the engaging groove 66 and which is the second engaging structure.

Further, when the catheter 69 is fixed to the organ 1, as illustrated in (2) of FIG. 11, the catheter 69 is first inserted in the organ 1 until the flange 68 abuts on the organ 1, and exposes the engaging groove 66 on the discharge side. Then, as illustrated in (3) of FIG. 11, a catheter fixture 70 is moved to the vicinity of the engaging groove 66 by bending the gripping part 71. Further, as illustrated in (4) of FIG. 11, the gripping part 71 grips the engaging groove 66 to engage and fix the catheter 69 and the catheter fixture 70 to the organ 1. In addition, similar to, for example, a treatment tool used for an endoscope, the catheter fixture 70 has an opening/closing and rotating mechanism which opens/closes and rotates the gripping part 71 positioned at the distal end from the outside of the body, and a bending mechanism which bends the holding part near the gripping part 71 from the outside of the body.

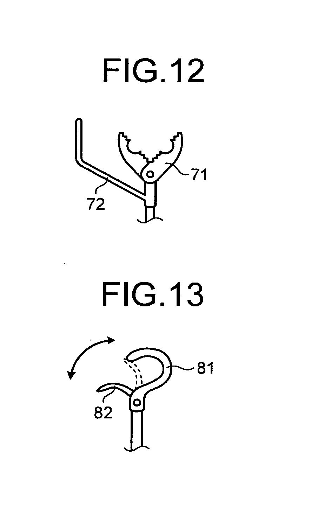

In addition, as illustrated in FIG. 12, a protecting part 72 may be provided which extends from the proximal end part of the gripping part 71 and which corresponds to the protecting part 27 according to the first embodiment. Further, as illustrated in FIG. 13, a hook 81 may be used instead of the gripping part 71. In this case, by engaging the hook 81 with the engaging groove 66 and closing an opening/closing part 82, the engaged state is maintained.

### Fifth Embodiment

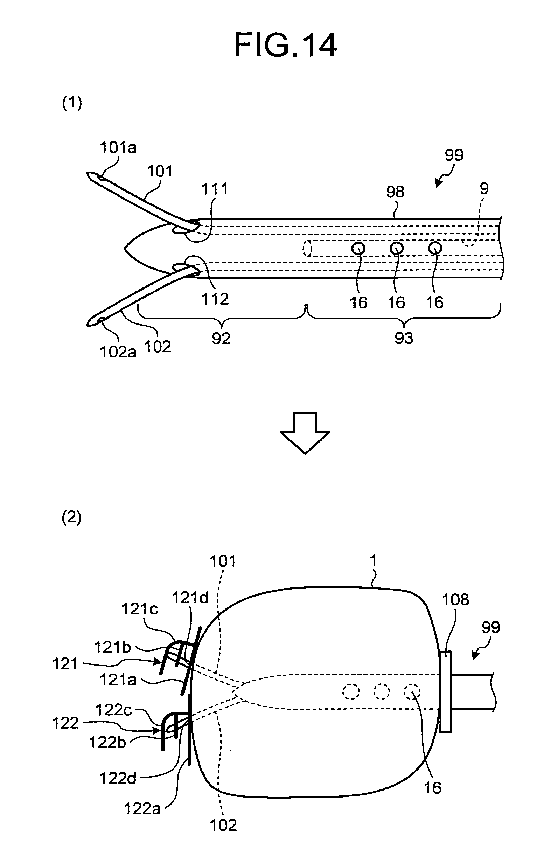

Next, a fifth embodiment of the present invention will be described. Although the above first engaging structure is one penetration hole 11 or one engaging groove 66 provided in the catheter body 8, with this fifth embodiment, as illustrated in (1) of FIG. 14, a plurality of engaging wire rods 101 and 102 are provided which extend from a distal end of a catheter 99 corresponding to the catheter 19 according to the first embodiment, and, as illustrated in (2) of FIG. 14, a plurality of engaging parts 121 and 122 are provided which engage with respective distal end parts of a plurality of engaging wire rods 101 and 102 and the catheter 99 is fixed to the organ 1 by engaging the engaging parts 121 and 122 with the engaging wire rods 101 and 102, respectively.

As illustrated in (1) of FIG. 14, in addition to the internal passage 9 in which a drug flows, a catheter body 98 of this catheter 99 allows penetration of the engaging wire rods 101 and 102 respectively in internal passages 111 and 112 provided in the catheter 99 along this internal passage 9. Penetration holes 101a and 102a are provided at each tip of each engaging wire rod 101 and 102.

In a state where each engaging wire rod 101 and 102 is accommodated in each internal passage 111 and 112, this catheter 99 is inserted until a flange 108 abuts on the organ 1 as illustrated in (2) of FIG. 14. Then, each engaging wire rod 101 and 102 accommodated in each internal passage 111 and 112 is projected and penetrates in the organ 1 to project outside the organ 1. Meanwhile, an opening at the distal end of each internal passage 111 and 112 diagonally opens in a radial direction, and each engaging wire rod 101 and 102 widens and projects outside the organ 1. Further, the opening at the distal end of each internal passage 111 and 112 is provided in a distal end part 92 corresponding to the distal end part 12 according to the first embodiment. Further, the drug discharge holes 16 are provided in a punching area 93 corresponding to the punching area 13 according to the first embodiment.

As illustrated in (2) of FIG. 14, each engaging part 121 and 122 which engages with each engaging wire rod 101 and 102 has flanges 121a and 122a which correspond to the slide bar 21 or flange 31 according to the first embodiment, lock parts 121b and 122b which correspond to the lock bar 24 according to the first embodiment, protecting parts 121c and 122c which correspond to the protecting part 27 according to the first embodiment and penetration holes 121d and 122d which correspond to the penetration hole 22 or penetration hole 32. After each engaging wire rod 101 and 102 projects, the tip of each engaging wire rod 101 and 102 passes in each penetration hole 121d and 122d, each lock part 121b and 122b passes in penetration holes 101a and 102a provided at the tip of each engaging wire rod 101 and 102 and each engaging wire rod 101 and 102 is extended from outside the body to fix the catheter 99 to the organ 1. Meanwhile, the first engaging structure is the penetration holes 101a and 102a, and the second engaging structure is the lock parts 121b and 122b.

With this fifth embodiment, each engaging wire rod 101 and 102 in the engaged state is extended and the catheter 99 is reliably and flexibly fixed to the organ 1. Further, the distal end side of the catheter 99 is fixed at a plurality of points in a dispersed manner, so that the catheter 99 can be more reliably fixed to the organ 1.

### Sixth Embodiment

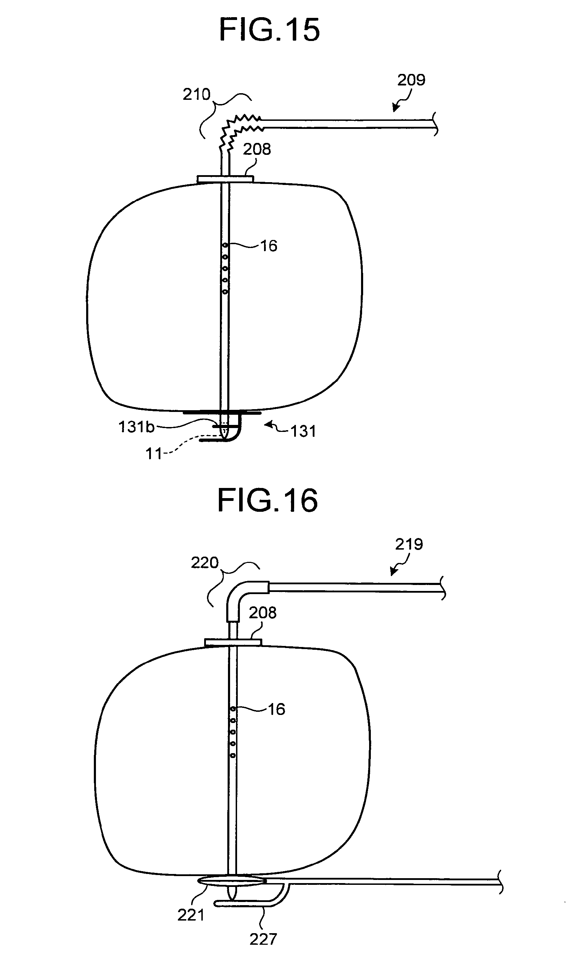

Next, a sixth embodiment of the present invention will be described. With this sixth embodiment, the insertion direction of the catheter with respect to the organ 1 can be changed based on an arrangement relationship of surrounding organs. That is, with this sixth embodiment, as illustrated in FIG. 15, a bent part 210 is provided on a proximal end part side of the drug administration holes 16. With a catheter 209 illustrated in FIG. 15, the bent part 210 adopting an accordion structure is provided on the proximal end side of a flange 208, and, in FIG. 15, the invasion direction of the catheter 209 is left and right directions, and the insertion direction of the catheter 209 to the organ 1 is up and down directions. In addition, at the distal end of the catheter 209, an engaging part 131 is provided which is the same as the engaging parts 121 and 122 illustrated in FIG. 14, such that the penetration hole 11 provided at the tip of the catheter 209 and a locking part 131b of the engaging part 131 engage.

In addition, as illustrated in FIG. 16, a bent part 220 of an L-shaped pipe may be provided instead of the bent part 210 adopting the accordion structure. Further, in FIG. 16, instead of the engaging part 131, a gripping part 221 and protecting part 227 respectively corresponding to the gripping part 71 and protecting part 72 illustrated in FIG. 12 grip and engage with the distal end of a catheter 219.

### Seventh Embodiment

Next, a seventh embodiment of the present invention will be described. Although, with the above first to sixth embodiments, all catheters adopt a structure of a closed distal end, with this seventh embodiment, a catheter having the open distal end is used.

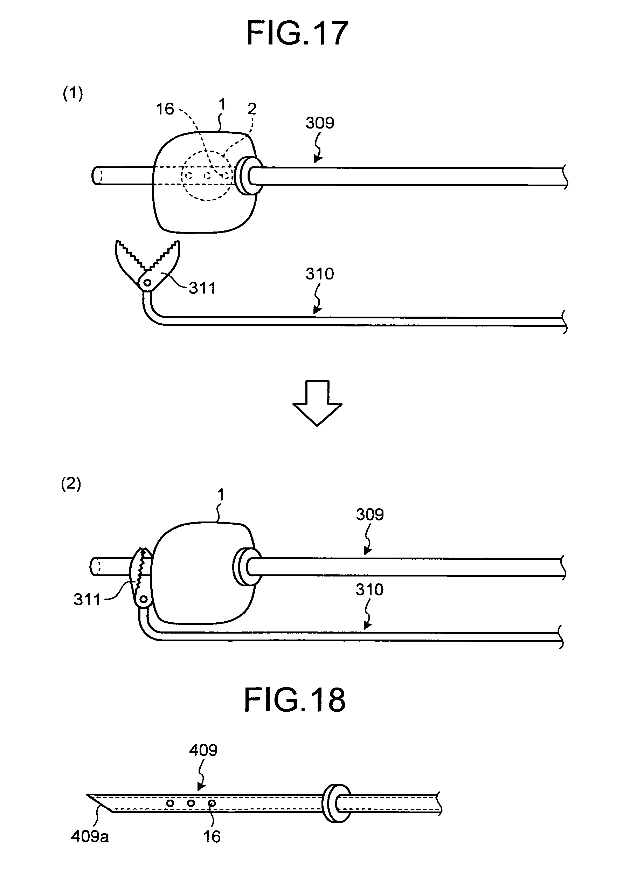

That is, as illustrated in FIG. 17, a catheter 309 forms a thin pipe having an open distal end and the diameter of about 1 mm, and, as illustrated in (1) of FIG. 17, the drug administration holes 16 are inserted in the organ 1 until the drug administration holes 16 are positioned in the tumor 2 of the organ 1. Then, as illustrated in (2) of FIG. 17, using a catheter fixture 310 having a gripping part 311 formed with a pair of forceps at the distal end, the gripping part 311 grips the distal end portion of the catheter 309 projecting from the organ 1 and further strongly sandwiches the distal end portion to crush and block the internal passage of the catheter 309, and the gripping part 311 maintains this gripping state. In this state, a drug which flows in the internal passage of the catheter 309 can be discharged from the drug discharge holes 16.

In addition, as illustrated in FIG. 18, an opening 409a formed by diagonally cutting the distal end and having a sharp shape may be used. In this case, even if the diameter of a catheter 409 is thick, it is possible to smoothly insert the catheter 409 in the organ 1.

Further, similar to the protecting part 72 illustrated in FIG. 12, a protecting part which extends from the proximal end part of the gripping part 311 to the vicinity of the distal end of the catheter 309 or catheter 409 may be provided to prevent the distal end of the catheter 309 or catheter 409 from damaging other organs or tissues.

With this seventh embodiment, the catheter having an open distal end can be used as is, so that a configuration is simple and the catheter and catheter fixture can be engaged at the same time when a drug administering catheter is formed.

### Eighth Embodiment

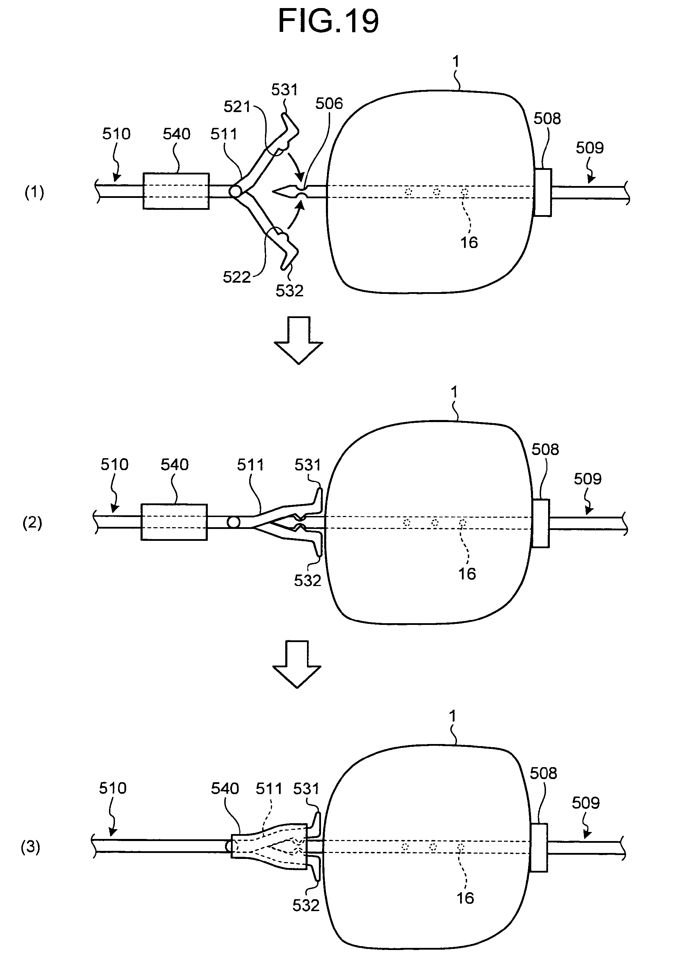

Next, an eighth embodiment of the present invention will be described. With this eighth embodiment, as illustrated in (1) of FIG. 19, a catheter 509 is provided at its distal end with an engaging groove 506 which is a first engaging structure instead of the penetration hole 11, and a catheter fixture 510 has a gripping part 511 having convex parts 521 and 522 and flanges 531 and 532 which are the second engaging structure which engages with this engaging groove 506. As illustrated in (1) of FIG. 19, the catheter 509 is first inserted in the organ 1 until a flange 508 abuts on the organ 1, and the engaging groove 506 of the distal end part is projected to the outside of the organ 1.

Further, in a state where the gripping part 511 provided at the distal end of the catheter fixture 510 is opened, the gripping part 511 is moved to the vicinity of the distal end of the catheter 509, the convex parts 521 and 522 are engaged with the engaging groove 506 and flanges 531 and 532 abut on the organ 1 (see (2) of FIG. 19). Further, as illustrated in (3) of FIG. 19, to maintain this engaged state, a cylindrical fixing part 540 inserted on the outer side from the proximal end side of a catheter fixture 510 is slid and fixed to the gripping part 511. By this means, the engaged state of the catheter 509 and catheter fixture 510 are reliably maintained, and the catheter 509 is fixed to the organ 1.

With this eighth embodiment, even when the diameter of the catheter 509 is small, it is possible to reliably fix the catheter 509 to the organ 1 without providing a penetration hole.

### Ninth Embodiment

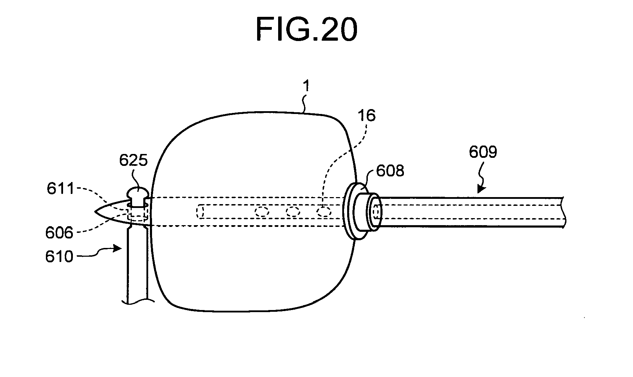

Next, a ninth embodiment of the present invention will be described. Although, with this ninth embodiment, as illustrated in FIG. 20, the same catheter 609 as in the eighth embodiment is used, the catheter 609 has a greater diameter than the eighth embodiment, so that it is possible to provide a penetration hole 611 in the distal end part of the catheter 609. A catheter fixture 610 is a fixing bar which serves as the slide bar 21 and lock bar 24, and has at the distal end part an engaging groove 606 which engages with a penetration hole 611 and an elastic ball 625 at the distal-most end part.

When the catheter 609 is inserted in the organ 1 until a flange 608 of the catheter 609 abuts on the organ 1, the distal end part projects outside the organ 1 and penetration hole 611 projects. The elastic ball 625 is elastically deformed and inserted in this penetration hole 611, and the engaging groove 606 engages with the penetration hole 611. By this means, the catheter 609 and catheter fixture 610 engage, and the catheter 609 is fixed to the organ 1.

In addition, similar to the protecting part 72 illustrated in FIG. 12, the catheter fixture 610 is provided with a protecting part which extends from the proximal end part of the engaging groove 606 to the vicinity of the distal end of the catheter 609 to prevent the distal end of the catheter 609 from damaging other organs and tissues.

With this ninth embodiment, it is possible to fix the catheter 609 to the organ 1 with a simple configuration.

In addition, although, with the above first to ninth embodiments, the tumor 2 inside the organ 1 is a drug administration target, the drug administration target is not limited to the tumor 2 and may be a site of lesion or blood vessel concentrating part such as the spleen. Reference Signs List

- 1: ORGAN
- 2: TUMOR
- 3: INSERTION ROUTE
- 4, 6: PROJECTING POSITION
- 5, 7: INSERTION POSITION
- 8, 98: CATHETER BODY
- 9, 111, 112: INTERNAL PASSAGE
- 10: TIP
- 11, 22, 28, 32, 45, 55, 101a, 102a, 121d, 122d, 611: PENETRATION HOLE
- 12, 62, 92: DISTAL END PART
- 13, 63, 93: PUNCHING AREA
- 14: MARK LINE
- 15: SCALE
- 16: DRUG ADMINISTRATION HOLE
- 17: FIXING MEMBER
- 18, 31, 68, 108, 121a, 122a, 208, 508, 531, 532, 608: FLANGE
- 19, 19a, 69, 99, 209, 219, 309, 409, 509, 609: CATHETER
- 20, 20a, 30, 40, 50, 70, 310, 510, 610: CATHETER FIXTURE
- 21: SLIDE BAR
- 23, 41, 51: SLIDER
- 24: LOCK BAR
- 25, 625: ELASTIC BALL
- 26, 66, 506, 606: ENGAGING GROOVE
- 27, 72, 121c, 122c, 227: PROTECTING PART
- 41a, 51a: CAP
- 42, 52: TUBE
- 43: WIRE
- 44, 54: LATCH PART
- 46, 56: HOLDING PART
- 47: RATCHET MECHANISM
- 53: STRETCHING PART
- 57: FLUID PUMP
- 58: DRUG ADMINISTRATION PUMP
- 71, 221, 311, 511: GRIPPING PART
- 81: HOOK
- 82: OPENING/CLOSING PART
- 101, 102: ENGAGING WIRE ROD
- 121, 122, 131: ENGAGING PART
- 121b, 122b, 131b: LOCK PART
- 210, 220: BENT PART
- 409a: OPENING
- 521, 522: CONVEX PART
- 540: FIXING PART
- M1: MARK
- P2, P11, P12, P13: POSITION
- La, Lb, Lc, La', Lb', Lc', L1, L2: DISTANCE
- C1: IN-VIVO OBSERVATION DEVICE
- C2: DISPLAY DEVICE
- C3: PROCESSING DEVICE

## Claims

1. A catheter kit comprising:
an elongated tubular catheter having at least one drug discharge hole in a side of the catheter, the catheter having a first engaging structure in a distal end part closer to a distal end side than the drug discharge hole; and
a catheter fixture that is separated from the catheter, the catheter fixture having a second engaging structure that engages with the first engaging structure, the catheter fixture being fixed to the distal end part of the catheter with when the first engaging structure and the second engaging structure engage, the catheter fixture being wider than a diameter of the catheter in a direction perpendicular to an axis of the catheter in a fixed state.

2. The catheter kit according to claim 1, wherein
the catheter is used to administer a drug to a drug administration target in an organ, and
when the catheter penetrates the organ and the catheter fixture is in a position to abut on the organ in a state where the catheter fixture is fixed to the distal end part, the drug discharge hole is provided in a position adjusted in advance such that the drug discharge hole is positioned inside the drug administration target.

3. The catheter kit according to claim 1, wherein the catheter has a flange in a position closer to a proximal end side than a position of at least one drug discharge hole.

4. The catheter kit according to claim 3, wherein
the catheter is used to administer a drug to a drug administration target in an organ, and
when the catheter penetrates the organ and the catheter fixture is in a position to abut on the organ in a state where the catheter fixture is fixed to the distal end part, the flange is provided in a position on the catheter adjusted in advance such that the flange abuts on the organ.

5. The catheter kit according to claim 1, wherein the catheter fixture has a protecting part that prevents a tip of the catheter and an organism from contacting, in a part positioned on an extension of an axis line on a distal end side of the catheter in a state where the catheter fixture is fixed to the distal end part of the catheter.

6. The catheter kit according to claim 1, wherein
the catheter is for puncturing an organ, and
the catheter fixture has a flange that abuts on the organ, in a part positioned closer to a proximal end side of the catheter than the second engaging structure in a state where the catheter fixture is fixed to the distal end part of the catheter.

7. The catheter kit according to claim 1, wherein
the catheter fixture includes a base member and a slider having the second engaging structure, and
when the catheter and the catheter fixture are in predetermined positions, the slider slides with respect to the base member such that the first engaging structure of the catheter and the second engaging structure of the catheter fixture engage.

8. The catheter kit according to claim 7, wherein the slider slides with respect to the base member according to a manipulation from an outside of a body.

9. The catheter kit according to claim 8, wherein the manipulation from the outside of the body is a manipulation of a wire for sliding the slider.

10. The catheter kit according to claim 8, wherein the manipulation from the outside of the body is a manipulation of moving a fluid for sliding the slider, with respect to the catheter fixture.

11. The catheter kit according to claim 1, wherein
the catheter fixture has an elongated wire rod,
the second engaging structure is a gripping part that grips the first engaging structure, and
the gripping part is provided at a distal end of the wire rod and is manipulated at a proximal end side of the wire rod.

12. The catheter kit according to claim 1, wherein
the catheter has a wire rod that stretches from a distal end part, and
the first engaging structure is provided in the wire rod.

13. The catheter kit according to claim 12, wherein
a plurality of wire rods are provided, and
the first engaging structure is provided in each of the plurality of wire rods.

14. The catheter kit according to claim 1, wherein, on a surface of the catheter, a pattern which serves as an indication to determine a punching position of the drug discharge hole is displayed.

15. The catheter kit according to claim 1, wherein the distal end part can be separated from the catheter.

16. The catheter kit according to claim 3, wherein the catheter can be separated at a distal end side of the flange.

17. The catheter kit according to claim 1, wherein the catheter has a bent part closer to a proximal end side than the drug administration hole.

18. A catheter kit comprising:
an elongated tubular catheter having at least one drug discharge hole in a side, having a first engaging structure in a distal end part closer to a distal end side than the drug discharge hole, and having a first flange that is a flange provided in a position closer to a proximal end side than a position of the drug discharge hole; and
a catheter fixture that is separated from the catheter, the catheter fixture having a second engaging structure that engages with the first engaging structure, and having a second flange that is a flange provided in a position closer to the distal end side than a position of the drug discharge hole.

19. The catheter kit according to claim 18, wherein the second engaging structure forms the second flange.

20. A catheter for puncturing an organ to administer a drug to a drug administration target inside the organ,
the catheter comprising an elongated tubular body, the body having at least one drug discharge hole in a side, the body having a flange closer to a proximal end side than the drug discharge hole, wherein
when the catheter punctures the organ along a route determined in advance and the flange is in a position to abut on the organ, the drug discharge hole is provided in a position generated in advance such that the drug administration hole is positioned inside the drug administration target.

21. A catheter comprising:
an elongated tubular catheter body having at least one drug discharge hole; and
at least one wire rod capable of being transitioned from a first state where the entire wire rod is accommodated in an internal passage inside the catheter body to a second state where a distal end part of the wire rod projects from the internal passage on a side closer to a distal end side than the drug discharge hole, the wire rod fixing the catheter body to a body tissue in the second state.

22. A catheter fixture, separated from a catheter for puncturing an organ to administer a drug to a drug administration target inside the organ, the catheter fixture comprising a second engaging structure that engages with a first engaging structure formed at a distal end part of the catheter, the catheter fixture being fixed to the distal end part of the catheter when the first engaging structure and the second engaging structure engage, the catheter fixture being wider than a diameter of the catheter in a direction perpendicular to an axis of the catheter in a fixed state.

23. A method of manufacturing a catheter to be inserted in an organ to administer a drug to a drug administration target inside the organ, the method comprising:
a target position acquiring step of acquiring positions of the organ and the drug administration target using an in-vivo observation device;
a determining step of determining an insertion route of the catheter to the organ, based on the positions of the organ and the drug administration target acquired in the target position acquiring step; and
a hole forming step of forming a drug discharge hole in a side of the catheter which meets the drug administration target, based on the positions of the organ and the drug administration target and the determined insertion route.

## Amended claims

### Amended claims under Art. 19.1 PCT

**1.** A catheter kit comprising:
an elongated tubular catheter having at least one drug discharge hole in a side of the catheter, the catheter having a first engaging structure in a distal end part closer to a distal end side than the drug discharge hole; and
a catheter fixture that is separated from the catheter, the catheter fixture having a second engaging structure that engages with the first engaging structure, the catheter fixture being fixed to the distal end part of the catheter with when the first engaging structure and the second engaging structure engage, the catheter fixture being wider than a diameter of the catheter in a direction perpendicular to an axis of the catheter in a fixed state.

**2.** The catheter kit according to claim 1, wherein
the catheter is used to administer a drug to a drug administration target in an organ, and
when the catheter penetrates the organ and the catheter fixture is in a position to abut on the organ in a state where the catheter fixture is fixed to the distal end part, the drug discharge hole is provided in a position adjusted in advance such that the drug discharge hole is positioned inside the drug administration target.

**3.** The catheter kit according to claim 1, wherein the catheter has a flange in a position closer to a proximal end side than a position of at least one drug discharge hole.

**4.** The catheter kit according to claim 3, wherein
the catheter is used to administer a drug to a drug administration target in an organ, and
when the catheter penetrates the organ and the catheter fixture is in a position to abut on the organ in a state where the catheter fixture is fixed to the distal end part, the flange is provided in a position on the catheter adjusted in advance such that the flange abuts on the organ.

**5.** The catheter kit according to claim 1, wherein the catheter fixture has a protecting part that prevents a tip of the catheter and an organism from contacting, in a part positioned on an extension of an axis line on a distal end side of the catheter in a state where the catheter fixture is fixed to the distal end part of the catheter.

**6.** The catheter kit according to claim 1, wherein
the catheter is for puncturing an organ, and
the catheter fixture has a flange that abuts on the organ, in a part positioned closer to a proximal end side of the catheter than the second engaging structure in a state where the catheter fixture is fixed to the distal end part of the catheter.

**7.** The catheter kit according to claim 1, wherein
the catheter fixture includes a base member and a slider having the second engaging structure, and
when the catheter and the catheter fixture are in predetermined positions, the slider slides with respect to the base member such that the first engaging structure of the catheter and the second engaging structure of the catheter fixture engage.

**8.** The catheter kit according to claim 7, wherein the slider slides with respect to the base member according to a manipulation from an outside of a body.

**9.** The catheter kit according to claim 8, wherein the manipulation from the outside of the body is a manipulation of a wire for sliding the slider.

**10.** The catheter kit according to claim 8, wherein the manipulation from the outside of the body is a manipulation of moving a fluid for sliding the slider, with respect to the catheter fixture.

**11.** The catheter kit according to claim 1, wherein
the catheter fixture has an elongated wire rod,
the second engaging structure is a gripping part that grips the first engaging structure, and
the gripping part is provided at a distal end of the wire rod and is manipulated at a proximal end side of the wire rod.

**12.** The catheter kit according to claim 1, wherein
the catheter has a wire rod that stretches from a distal end part, and
the first engaging structure is provided in the wire rod.

**13.** The catheter kit according to claim 12, wherein
a plurality of wire rods are provided, and
the first engaging structure is provided in each of the plurality of wire rods.

**14.** The catheter kit according to claim 1, wherein, on a surface of the catheter, a pattern which serves as an indication to determine a punching position of the drug discharge hole is displayed.

**15.** The catheter kit according to claim 1, wherein the distal end part can be separated from the catheter.

**16.** The catheter kit according to claim 3, wherein the catheter can be separated at a distal end side of the flange.

**17.** The catheter kit according to claim 1, wherein the catheter has a bent part closer to a proximal end side than the drug administration hole.

**18.** A catheter kit comprising:
an elongated tubular catheter having at least one drug discharge hole in a side, having a first engaging structure in a distal end part closer to a distal end side than the drug discharge hole, and having a first flange that is a flange provided in a position closer to a proximal end side than a position of the drug discharge hole; and
a catheter fixture that is separated from the catheter, the catheter fixture having a second engaging structure that engages with the first engaging structure, and having a second flange that is a flange provided in a position closer to the distal end side than a position of the drug discharge hole.

**19.** The catheter kit according to claim 18, wherein the second engaging structure forms the second flange.

**20.** (Amended) The catheter kit according to claim 1, wherein the catheter has a flange closer to a proximal end side than the drug discharge hole, and
when the catheter punctures the organ along a route determined in advance and the flange is in a position to abut on the organ, the drug discharge hole is provided in a position generated in advance such that the drug administration hole is positioned inside the drug administration target.

**21.** (Amended) The catheter kit according to claim 1, wherein
the catheter has at least one wire rod capable of being transitioned from a first state where the entire wire rod is accommodated in an internal passage inside the catheter body to a second state where a distal end part of the wire rod projects from the internal passage on a side closer to a distal end side than the drug discharge hole, the wire rod fixing the catheter body to a body tissue in the second state.

**22.** (Amended) The catheter fixture of the catheter kit according to claim 1.

**23.** A method of manufacturing a catheter to be inserted in an organ to administer a drug to a drug administration target inside the organ, the method comprising:
a target position acquiring step of acquiring positions of the organ and the drug administration target using an in-vivo observation device;
a determining step of determining an insertion route of the catheter to the organ, based on the positions of the organ and the drug administration target acquired in the target position acquiring step; and
a hole forming step of forming a drug discharge hole in a side of the catheter which meets the drug administration target, based on the positions of the organ and the drug administration target and the determined insertion route.
Claims 20, 21, 22 are changed to clarify the invention. Claims 1 to 19, 23 are unchanged.
In the attached sheet, claim 20, claim 21, claim 22 are amended. The other claims are retained unchanged.
